# EUROPÄISCHE PATENTSCHRIFT

(11) **EP 1 337 223 B1**
(45) Veröffentlichungstag und Bekanntmachung des Hinweises auf die Patenterteilung: **22.02.2006**
(21) Anmeldenummer: 01998151.3
(22) Anmeldetag: 28.11.2001
(51) Int. Cl.: A61K 6/10

(54) **DENTALMASSEN MIT VERRINGERTER TEMPERATURSENSIBILITÄT**
DENTAL COMPOUNDS WITH REDUCED TEMPERATURE SENSITIVITY
PRODUITS DENTAIRES DE SENSIBILITE REDUITE A LA TEMPERATURE

(30) Priorität: 28.11.2000 DE 10058846
(43) Veröffentlichungstag der Anmeldung: 27.08.2003
(73) Patentinhaber: 3M ESPE AG, 82229 Seefeld (DE)
(72) Erfinder: ECKHARDT, Gunther, 06231 Bad Dürrenberg (DE); LECHNER, Günther, 82237 Wörthsee (DE); WANEK, Erich, 86916 Kaufering (DE); ROMBACH, Andreas, 82211 Herrsching (DE)
(86) Internationale Anmeldenummer: PCT/EP2001/013852
(87) Internationale Veröffentlichungsnummer: WO 2002/043670

(56) Entgegenhaltungen:
- DE-A- 10 018 918
- DE-A- 19 942 459
- US-A- 3 453 242
- US-A- 5 130 348

## Beschreibung

Die Erfindung betrifft die Verwendung von N-Alkylaziridinepolyethem zur Vermindnung der Temperatur von Dentalmassen hinsichtlich Viskosität, wie in beansprucht in dem Ansprüchen.

Die durch chemische Reaktionen aushärtenden dentalen Abformmassen, beispielsweise auf der Grundlage von Aziridinopolyethem, A-Silikonen und C-Silikonen, bestehen üblicherweise aus einer Basis- und einer Katalysatorkomponente, die getrennt voneinander gelagert und über Mischsysteme oder auch per Hand vor der Applikation angemischt worden.

Nachteilig an den bisher bekannten dentalen Abformmassen auf der Grundlage von N-Alkylaziridinopolyethern, beispielsweise solchen, die aus der US 3453242 oder der DE 197 40 234 A1 bekannt sind, ist der Umstand, dass die Abformmassen bei Temperaturen unter 15°C eine sehr feste Konsistenz annehmen, die eine Verarbeitung erschweren oder unmöglich machen.

Erwärmt man die Abformmassen auf Temperaturen von 16 bis 23° C, können sie verarbeitet werden.

Falls jedoch eine schnelle Verarbeitung kalter Abformmassen gewünscht ist, kommt es wegen der sehr hohen Viskosität zu erheblichen Dosier- und Mischproblemen. Das kann im Extremfall zum Versagen eines automatischen Mischgerätes führen.

Die Verarbeitbarkeit relativ kalter Abformmassen hat für die praktische Durchführung der Abformung bei hohen Raumtemperaturen besondere Bedeutung, da durch eine Vorkühlung die Verarbeitungszeit verlängert werden kann.

Außerdem ist es durchaus üblich, zur Verlängerung der Haltbarkeit von reaktiven Abformmassen diese im Kühlschrank zu lagern.

In diesen Fällen erweist sich eine ausgeprägte Temperatursensibilität der Viskosität als besonders nachteilig.

Die unerwünschten Erscheinungen einer ausgeprägten Temperatursensibilität können beispielsweise durch Zumischen von örgänischen Verbindungen, wie Kohlenwasserstoffen, Polyolen und Estern vermindert werden.

Allerdings verändern diese Substanzen einige Eigenschaften, wie die elastische Verformbarkeit, negativ. Sie können aber auch nach der Aushärtung aus der Abformung austreten und die weitere Verarbeitung der Abformung behindem.

Der Zusatz solcher Substanzen kann deshalb nur in einem begrenzten Konzentrationsbereich erfolgen, wobei die zulässigen Konzentrationen meist noch keine wirksame Verringerung der Temperatursensibilität ermöglichen.

Es bestand demnach die Aufgabe, Dentalmassen zu formulieren, die eine nur geringe Temperatursensibilität hinsichtlich der Viskosität aufweisen und im ausgehärteten Zustand für den Gebrauch im Dentalbereich ausreichende mechanische Eigenschaften besitzen.

Diese Aufgabe wurde gelöst durch die Verwendung von N-Alkylaziridinopolyethern, denen Polyetherteil 10 bis 250, bevorzugt 126 bis 250 und besonders bevorzugt 180 bis 250 Methylseitengruppen pro 1.000 C- und O-Atome besitzt, wobei die zahlenmittleren Molmassen der N-Alkylaziridinopolyether im Bereich von 1.000 bis 25.000, bevorzugt 2.000 bis 18.000 und besonders bevorzugt von 3.000 bis 15.000 g/mol liegen,

Allgemein ist es bevorzugt, wenn der Polyetherteil des N-Alkylaziridinopolyethers überwiegend aus Propylenoxideinheiten besteht.

Die in dieser Schrift angegebenen Molmassen sind zahlenmittlere Molmassen, wie sie durch Endgruppenanalyse oder mittels Osmometrie bestimmt werden können.

Die Begriffe "umfassend" oder "enthaltend" sollen im Rahmen dieser Anmeldung stets eine nicht-abgeschlossene Aufzählung einleiten.

Der Umstand, dass das Wort "ein" vor Nennung eines Merkmals verwendet wird, schließt nicht aus, dass die genannten Merkmale mehrmals vorhanden sein können, im Sinne von "mindestens ein".

Bevorzugt eingesetzte Vertreter der erfindungsgemäßen N-Alkylaziridinopolyether sind Homo- und Mischpolymerisate des Propylenoxids, die durch nachfolgende Reaktionen mit einer bis zehn, bevorzugt einer bis vier, besonders bevorzugt zwei bis drei N-Alkylaziridinogruppen pro Molekül ausgestattet sind.

Die diesen N-Alkylaziridinopolyethern zugrundeliegenden Polyetherpolyole können beispielsweise durch Homo- oder Copolymerisation von Propylenoxid unter Verwendung niedermolekularer Starteralkohole und geeigneter Katalysatoren hergestellt werden.

Als besonders geeignet erweisen sich solche Homo- und Copolymerisate, die unter Verwendung von Zinkhexacyanocobaltat-Katalysatoren hergestellt wurden. Diese Technologie ist in zahlreichen Patenten, beispielsweise US 4,355,188; US 4,721,818; US 4,843,054; US 5,364,973 beschrieben.

Die Anzahl der OH-Gruppen der Polyetherpolyole wird bevorzugt durch die Wahl des Starteralkohols festgelegt. So werden beispielsweise zur Erzielung von Polyetherdiolen Ethylenglykol oder Polyethylenglykol und zur Erzielung von Polyethertriolen Trimethylolpropan oder Glycerin als Starteralkohol eingesetzt.

Als Comonomere kommen bevorzugt Ethylenoxid und/oder Tetrahydrofuran in Betracht.

Typische Vertreter der erfindungsgemäßen N-Alkylaziridinopolyether können aus Polypropylenoxiddiolen oder Polypropylenoxidtriolen hergestellt werden, wie sie von der Firma Bayer AG unter der Bezeichnung Acclaim® in einem Molmassenbereich von 2.000 bis 12.000 g/mol angeboten werden.

Mit Vorteil sind ebenfalls von der Firma Bayer AG (Lyondell) vertriebene Copolymerpolyole aus Ethylenoxid und Propylenoxid der Acclaim®-Reihe einsetzbar.

Aus den Polyetherpolyolen und beispielsweise den Polyetherdiolen oder Polyethertriolen können in an sich bekannter Weise die N-Alkylaziridinopolyether hergestellt werden, beispielsweise über Chloroxalate oder Chlorformiate, wobei in manchen Fällen aus den Chloroxalaten oder Chloroformiaten intermediär aktivierte Amide, wie Imidazolide verwendet werden. Als Aziridinokomponente kommen beispielsweise Aziridinoethanol oder andere hydroxy- oder aminofunktionelle Aziridinoverbindungen in Frage (H. Bestian in Houben-Weyl Bd. 11/2, S. 272 ff.). Ein anderer Weg besteht darin, Aziridin an aktivierte Doppelbindungen anzulagern, wie es in der US 3,453,242, Beispiel 13 beschrieben ist.

Vorzugsweise umfassen die Dentalmassen nach der Mischung der Basiskomponente mit der Katalysatorkomponente, jeweils bezogen auf 100 Masseteile:
(A) 30 bis 97, bevorzugt 40 bis 89, besonders bevorzugt 45 bis 80,5 Teile von mindestens einem N-Alkylaziridinopolyether, dessen Polyetherteil 10 bis 250, bevorzugt 126 bis 250, besonders bevorzugt 180 bis 250 Methylseitengruppen pro 1.000 C- und O-Atome besitzt, wobei die zahlenmittleren Molmassen der N-Alkylaziridinopolyether im Bereich von 1.000 bis 25.000, bevorzugt 2.000 bis 18.000 und besonders bevorzugt von 3.000 bis 15.000 g/mol liegen,
(B) 1 bis 10, bevorzugt 1 bis 5, besonders bevorzugt 1,5 bis 3 Teile von Startersubstanzen, die geeignet sind, die Aushärtung der N-Alkylaziridinopolyether zu bewirken,
(C) 1 bis 35, bevorzugt 5 bis 25, besonders bevorzugt 8 bis 20 Teile von organischen Verdünnungsmitteln,
(D) 1 bis 50, bevorzugt 5 bis 40, besonders bevorzugt 10 bis 30 Teile von Modifikatoren.

Vor der Mischung sind die Bestandteile (A) bis (D) auf die Basiskomponente und die Katatysatorkomponente derart aufgeteilt, dass der Bestandteil (A) vollständig in der Basiskomponente und der Bestandteil (B) vollständig in der Katalysatorkomponente enthalten ist, wogegen die Bestandteile (C) und (D) anteilig, beispielsweise im Verhältnis 20 : 80 bis 80 : 20 in beiden Komponenten vorliegen können.

Das Mischungsverhältnis kann in einem weiten Bereich über die Zusammensetzung der beiden Komponenten eingestellt werden, wobei sich Mischungsverhältnisse Katalysatorkomponente zu Basiskomponente von 1 : 1 bis 1 : 5 als besonders praktikabel erwiesen haben.

Die Dosierung der beiden Komponenten kann nach Sicht (Stranglängenvergleich), nach Gewicht, über vordosierte Packungseinheiten und nachfolgende Handanmischung, aus Doppelkammerkartuschen mit statischem Mischrohr oder mittels Mischgeräten aus direkt oder mit Schlauchbeuteln befüllten Kartuschen mit nachgeschaltetem statischen oder dynamischen Mischer erfolgen.

Die Anteile der einzelnen Bestandteile (A) bis (D) sind innerhalb der angegebenen Grenzen so einzustellen, dass eine günstige Verarbeitbarkeit hinsichtlich Mischungsverhältnis und Fließverhalten gewährleistet ist und die Kriterien für die gewünschte Verarbeitungszeit, die Aushärtungszeit und die mechanischen Eigenschaften der ausgehärteten Masse eingehalten werden.

Bestandteil (A) enthält die erfindungsgemäßen N-Alkylaziridinopolyether. Die Verwendung von Mischungen von N-Alkylaziridinopolyethern mit unterschiedlichen Molmassen und Aziridinoäquivalentmassen ist möglich und kann zur Einstellung der Eigenschaften der Dentalmassen genutzt werden.

Als Startersubstanz gemäß Bestandteil (B) der gemischten Zubereitung kommen die Verbindungen in Betracht, die die nachfolgend aufgeführten Kriterien hinsichtlich Abbindegeschwindigkeit und resultierenden Elastomereigenschaften erfüllen.

So sind für die Verwendung in zweikomponentigen Abformmassen, die auf den vorstehend beschriebenen Polyetherderivaten beruhen, erfahrungsgemäß solche Startersubstanzen geeignet, die eine Aushärtung der gemischten Zubereitung in einem Zeitraum von 1 bis 20 Minuten zu einem elastischen Festkörper ermöglichen, wobei dieser Festkörper eine Reißfestigkeit nach DIN/EN 4823 von mindestens 0,7 MPa, eine Reißdehnung nach DIN/EN 4823 von mindestens 70 % und eine Shore A-Härte DIN 53505 von mindestens 20, jeweils gemessen nach einer Lagerzeit von 24 Stunden bei 23° C und 50 % relativer Luftfeuchtigkeit, besitzt.

Es können solche Starter bzw. Startersysteme verwendet werden, die eine einfache Einstellung des Aushärtungsverlaufs zulassen und die reproduzierbare Erreichung der erforderlichen Niveaus der mechanischen Eigenschaften ermöglichen.

Für den Verarbeiter sind insbesondere zwei Kennwerte im Rahmen des Aushärtungsverlaufes von Interesse: die Verarbeitungszeit und die Zeit bis zur Erreichung der Weiterverarbeitbarkeit der aushärtenden Masse.

Als Verarbeitungszeit wird hierbei der Zeitraum zwischen dem Beginn der Vermischung der Komponenten und der beginnenden Aushärtung der gemischten Zubereitung bei Raumtemperatur verstanden. Dieser Polymerisationsbeginn wird als der Zeitpunkt angesehen, bei dem eine gemischte Zubereitung aus der plastischen Phase in die elastische Phase übergeht und deutliche Veränderungen, wie Hautbildung, Fädenziehen und stark verringerte Fließfähigkeit zeigt.

Die gemischte Zubereitung ist bis kurz vor diesem Zeitpunkt noch verarbeitbar und zeigt das erforderliche Anfließen an die jeweiligen Substrate.

Üblicherweise wünscht der Verarbeiter eine Verarbeitungszeit von mehr als 30 Sekunden bei Raumtemperatur, um genügend Zeit zu haben, die gegebenenfalls mehrfache Platzierung und Dosierung der aushärtenden Masse sowie Korrekturmaßnahmen ausführen zu können. Hierbei erweist sich die Einstellung langer Verarbeitungszeiten als besonders schwierig.

Die Forderung nach langen Verarbeitungszeiten ist meist verbunden mit dem Wunsch, die Weiterverarbeitbarkeit der aushärtenden Masse nach einem möglichst kurzen Zeitraum möglich zu machen.

Für viele Einsatzfälle ist es erwünscht, Verarbeitungszeiten von 2 bis 4 Minuten zu erreichen und die Weiterverarbeitbarkeit in weniger als 7 Minuten nach Mischungsende zu gewährleisten. Als Weiterverarbeitung wird beispielsweise im Dentalbereich die Entnahme des ausgehärteten Abdrucks aus dem Mund des Patienten mit nachfolgender Desinfektion verstanden. Dies darf nicht zu früh geschehen, da ansonsten durch die noch nicht fertig abgebundene Masse die Präzision des Abdrucks zerstört wird.

Unter Verwendung von speziellen Trisalkylsulfoniumsalzen, wie dem Polymerisationsinitiator, der in der US 4,167,618, Beispiel 84 beschrieben ist, sind die Kriterien der Härtungsgeschwindigkeit und der Eigenschaften des elastischen Festkörpers der vorliegenden Erfindung erreichbar.

In der DE 100 18 918 A1 werden Starter beschrieben, die der Katalysatorkomponente einen lediglich geringen Säuregrad verleihen und die eine gut einstellbare, relativ lange Verarbeitungszeit nach erfolgter Mischung von Basiskomponente und Katalysatorkomponente ermöglichen. Startersysteme dieses Typs sind geeignet, die erfindungsgemäßen Basispasten in der notwendigen Geschwindigkeit auszuhärten. Durch ihre Verwendung sind die gewünschten Eigenschaften des elastischen Festkörpers erreichbar.

Die DE 199 42 459 A1 beschreibt Elastomermassen mit verbesserter Kätalysatorkomponente, die sich durch eine erhöhte Dehnbarkeit auszeichnen. Gemäß dieser Erfindung werden Borsäurekomplexe als Starter eingesetzt. Diese Starter sind für die Aushärtung der N-Alkylaziridinopolyether gemäß vorliegender Erfindung besonders gut geeignet.

Als organisches Verdünnungsmittel entsprechend Bestandteil (C) können Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle- Ester von mehrwertigen Säuren, wie Phthalsäure oder Zitronensäure, oder Ester oder Amide von Alkylsulfonsäuren und Arylsulfonsäuren verwendet werden.

Der Katalysatorkomponente wie auch der Basiskomponente werden Modifikatoren entsprechend dem Bestandteil (D) zugesetzt.

Diese Modifikatoren sind meist feinteilige Füllstoffe, wie Alumosilikate, Fällungskieselsäuren, Quarzmehl, Wollastonit, Glimmermehl und Diatomeenerde sowie Farbstoffe und Pigmente, Thixotropiemittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und Geschmacksstoffe.

Mit besonderem Vorteil werden die Zubereitungen gemäß der Erfindung bei der zahnmedizinischen und zahntechnischen Abformung eingesetzt.

Bei der zahnmedizinischen Abformung erweist sich das gute Anfließverhalten an den feuchten Zahn und das feuchte Zahnfleisch sowie die Unempfindlichkeit der Präzision der Abformung gegenüber Speichel und Blut als großer Vorteil.

Die Erfindung wird durch die nachfolgenden Beispiele näher beschrieben.

### Herstellungsbeispiele Dentalmassen

Mit Hilfe von Laborknetem wurden die beschriebenen Katalysatorkomponenten K1 bis K4 im 100 g-Maßstab hergestellt. Die Herstellung der Basiskomponenten, die in der Tabelle 1 beschrieben sind, erfolgte im 500 g-Maßstab.

In Tabelle 2 sind die Mischungen zusammengestellt, die unter Verwendung der beschriebenen Katalysatorkomponenten und den in der Tabelle 1 beschriebenen Basiskomponenten im jeweils angegebenen Gewichtsverhältnis untersucht wurden.

Die Mischungen gemäß Tabelle 2 wurden durch Anspateln auf den Mischblock innerhalb von 30 Sekunden zubereitet und zur Bestimmung der in Tabelle 3 zusammengestellten Eigenschaften eingesetzt.

### Herstellung der Katalysatorkomonenten

### Katalysatorkomponente K1

In einem Laborkneter wurden 44 g Acetyltributylcitrat vorgelegt und 22 g β-(S-Stearyl-S-ethylsulfonium)butyronitrilfluoroborat (hergestellt gemäß US 4,167,618, Beispiel 84) eingelöst. In diese Mischung wurden 10 g Diatomeenerde und 24 g pyrogener Kieselsäure (HDK H 2000, Fa. Wacker) eingearbeitet.

### Katalysatorkomponente K2 (gemäß DE 100 18 918 A1)

In einem Laborkneter wurden 61,1 g eines Poly(-ethylenoxid, -propylenoxid)-diols mit einer Molmasse von 3.000 g/Mol (Fa. Bayer AG, Acclaim® 3201) vorgelegt und schrittweise 21 g einer hydrophobierten Fällungskieselsäure (Sipernat® D 17, Fa. Degussa) zugegeben.

9,9 g p-Toluolsulfonsäure-Monohydrat wurden in 5 g destilliertem Wasser gelöst und der pastenförmigen Mischung zugesetzt. Nach Homogenisierung erfolgte die Zugabe einer Paste bestehend aus 2 g Zinkoxid und 1 g Poly(-ethylenoxid, -propylenoxid)-diol (Fa. Bayer AG, Acclaim® 3201) mit einer Molmasse von 3.000 g/mol. Die Katalysatorkomponente wurde nach der letzten Zugabe noch eine Stunde geknetet.

### Katalysatorkomponente K3 (gemäß DE 199 42 459 A1)

In einem Laborkneter wurden 25 g hydrophobierte. Fällungskieselsäure (Sipemat® D 17, Fa. Degussa) in 40 g eines Polypropylenoxiddiols mit einer Molmasse von 2.000 g/mol (Fa. Bayer AG, Acclaim® 2200) eingearbeitet. Dieser pastenförmigen Mischung wurde die Lösung einer Komplexverbindung, hergestellt aus 1,0 g Borsäure und 6,5 g 5-Brom-Salicylalkohol, in 27,5 g Polypropylenoxid-diol mit einer Molmasse von 2.000 g/mol (Fa. Bayer AG, Acclaim® 2200) zugegeben und die Paste eine Stunde geknetet.

### Katalysatorkomponente K4 (gemäß DE 199 42 459 A1)

In einem Laborkneter wurden 25 g hydrophobierte Fällungskieselsäure (Sipemat® D 17, Fa. Degussa) in 29,5 g Polypropylenoxiddiol mit einer Molmasse von 2.000 g/mol (Fa. Bayer AG, Acclaim® 2200) eingearbeitet. Dieser pastenförmigen Mischung wurde eine Lösung aus 2,4 g Borsäure, und 12,4 g 5-Chlorsalicylalkohol in 30,7 g Poly(-ethylenoxid, -propylenoxid)-diol mit einer Molmasse von 3.000 g/mol zugegeben (Fa. Bayer AG, Acclaim® 3201) und die Paste eine Stunde geknetet.

**Tabelle 1: Zusammensetzung von erfindungsgemäßen Basiskomponenten**

| Bestandteil | | B1 | B2 | B3 | B4 | B5 | B6 |
|---|---|---|---|---|---|---|---|
| | | Gew.-% | | | | | |
| (A) | Bisaziridinopolypropylenoxid mit 250 Methylseitengruppen pro 1.000 C- und O-Atome im Polyetherteil und einer Molmasse von 12.400 g/mol, hergestellt aus Polypropylenoxiddiol (Acclaim® 12200, Fa. Bayer) analog US 3,453,242, Beispiel 13 | 53,0 | 32,7 | 51,6 | 61,3 | 65,8 | 66,0 |
| (A) | Bisaziridinopolypropylenoxid mit 250 Methylseitengruppen pro 1.000 C- und O-Atome im Polyetherteil und einer Molmasse von 8.300 g/mol, hergestellt aus Polypropylenoxiddiol (Acclaim® 8200, Fa. Bayer) analog US 3,453,242, Bsp. 13 | - | 19,2 | - | - | - | - |
| (A) | Trisaziridinopolypropylenoxid mit 250 Methylseitengruppen pro 1.000 C- und O-Atome im Polyetherteil und einer Molmasse von 6.500 g/mol, hergestellt aus Polypropylenoxidtriol (Acclaim® 6300, Fa. Bayer) analog US 3,453,242, Bsp.13 | 5,1 | 3,5 | 4,0 | - | 6,2 | - |
| (A) | Bisaziridinopoly(propylenoxid, ethylenoxid) mit 180 Methylseitengruppen pro 1.000 C-und O-Atome im Polyetherteil und mit einer Molmasse von 3.400 g/mol, hergestellt aus einem statistischen Poly(propylen-oxid, ethylenoxid)-diol (Acclaim® 3201, Fa. Bayer) analog US 3,453,242, Beispiel 13 | - | 2,0 | 2,0 | - | - | - |
| (C) | Dibenzyltoluol | 9,7 | 10,3 | - | 4,5 | - | - |
| (C) | Polypropylenoxiddiol mit einer Molmasse von 2.000 g/mol (Acclaim® 2200, Fa. Bayer) | - | - | 10,2 | 6,3 | - | - |
| (D) | Hydrierter Rindertalg | 16,2 | 16,4 | 16,9 | 13,1 | 10,0 | 11,0 |
| (D) | Diatomeenerde (Celatom® MW 25, Fa. CHEMAG) | 16,0 | 15,9 | 15,3 | 14,8 | 18,0 | 23,0 |

**Tabelle 2: Erfindungsbeispiele**

| | Beispiele-Nr. | | | | | | | |
|---|---|---|---|---|---|---|---|---|
| | 1 | 2 | 3 | 4 | 5 | 6 | 7 | 8 |
| Basiskomponente gemäß Tabelle 1 | B1 | B1 | B2 | B3 | B4 | B4 | B5 | B6 |
| Katalysatorkömponente | K1 | K4 | K4 | K2 | K1 | K4 | K3 | K3 |
| Mischungsverhältnis nach Gewicht Katalysatorkomponente : Basiskomponente | 1 : 5 | 1 : 5 | 1 : 5 | 1 : 5 | 1 : 5 | 1 : 5 | 1 : 2 | 1 : 2 |

**Tabelle 3: Verarbeitungsverhalten und erreichte mechanische Eigenschaften der Zubereitungen gemäß den Beispielen 1 bis 8 (Tabelle 2)**

| Eigenschaft | Beispiele-Nr. | | | | | | | |
|---|---|---|---|---|---|---|---|---|
| | 1 | 2 | 3 | 4 | 5 | 6 | 7 | 8 |
| Verarbeitungszeit (23°C) [sec] nach EN 24823 | 155 | 105 | 110 | 185 | 160 | 125 | 130 | 150 |
| Aushärtungsende^{a)} (23°C) [sec] | 420 | 400 | 390 | 560 | 390 | 380 | 450 | 530 |
| Shore-A-Härte nach 24 h gemäß DIN 53505 | 48 | 49 | 47 | 45 | 44 | 45 | 44 | 42 |
| Reißdehnung [%] gemäß DIN/EN 4823 | 75 | 95 | 100 | 80 | 90 | 105 | 125 | 230 |
| Reißfestigkeit [MPa] gemäß DIN/EN 4823 | 0,80 | 1,08 | 1,23 | 0,85 | 0,95 | 0,95 | 1,10 | 1,05 |

| | | | | | | | | |
|---|---|---|---|---|---|---|---|---|
| ^{a)} Als Aushärtungsende wird der Zeitpunkt definiert, bei dem ein elastischer Festkörper vorliegt, der gemäß EN24823 ein elastisches Rückstellvermögen von 96,5 bis 100% besitzt. In der Praxis entspricht das dem. Zeitpunkt, bei dem der Festkörper keine durch Betasten wahrnehmbare Oberflächenktebrigkeit mehr besitzt. | | | | | | | | |

### Vergleichsbasiskomponente (VB1)

Als Vergleich zu den erfindungsgemäßen Basiskomponenten wurde eine Basiskomponente gewählt, wie sie von der DE 100 01 747 A1 als Basiskomponente B2 umfasst ist.

Die Vergleichsbasiskomponente enthält:

| **Bestandteil** | **Gew.-%** |
|---|---|
| Bis-Aziridinopolyether, Mₙ = 6100 g/mol, Einbauverhältnis Ethylenoxid- zu Tetrahydrofuran-Einheiten von 1 : 3,5, Restgehalt an oligomeren cyclischen Polyethern von 0,25 Gew.-% (Fa. ESPE) | 51,6 |
| Dibenzyltoluol | 1,2 |
| Synthetisches Triglycerid mit einem Stearoylanteil von 75 Gew.-% | 18,9 |
| Bisacetylpolyether, Mₙ = 5950 g/mol, Einbauverhältnis Ethylenoxid- zu Tetrahydrofuran-Einheiten von 1 : 3,6, Restgehalt an oligomeren cyclischen Polyethem von 0,29 Gew.-%, hergestellt durch Umsetzung des entsprechenden Mischpolyetherdiols mit Acetanhydrid gemäß EP 0 421 371 B1, Herstellungsbeispiel 4 | 20,3 |
| Diatomeenerde, pH-Wert der 5 %-igen wässrigen Dispersion: 9,4 | 7,7 |
| Laurylimidazol | 0,3 |

### Bestimmung der Temperatursensibilität

Die Bestimmung der Temperatursensibilität erfolgte durch Penetrationsbestimmung in Abhängigkeit von der Temperatur in Anlehnung an die DIN ISO 2137, wobei die nachstehend beschriebenen Anpassungen an das Messproblem realisiert wurden:

Die zu charakterisierende Paste wurde blasenfrei bis zum oberen Rand in ein zylindrisches Gefäß mit einem Innendurchmesser von 36 mm und einer Tiefe von 32 mm eingefüllt und das gefüllte Gefäß über einen Zeitraum von 24 h bei der gewählten Temperatur belassen. Dann wurde das temperierte, mit der Paste befüllte Gefäß aus dem Temperierschrank entnommen und sofort in eine Zugprüfmaschine der Firma Zwick eingespannt. Beim Meßvorgang wurde ein halbkugetförmig auslaufender zylindrischer Körper mit einem Außendurchmesser von 27 mm mit einem Vorschub von 15 mm/Minute in die temperierte Paste eingedrückt. Es wurde der Anstieg des Gegendruckes gegen die Vorschubzeit registriert. Für den Vergleich der Deformierbarkeit der Paste in Abhängigkeit von ihrer Temperatur wurde der Wert des Gegendrucks verwendet, der 15 Sekunden nach Versuchsbeginn erreicht wird.

In Tabelle 4 sind diese Werte des Gegendruckes der Basiskomponenten B1 bis B6 und der Vergleichsbasiskomponente VB1 für die Temperaturstufen +23° C, +10° C, 0° C, -10° C, -20° C dargestellt.

Aus Tabelle 4 ist zu erkennen, dass der Anstieg des Gegendrucks mit fallender Temperatur bei den erfindungsgemäßen Beispielen wesentlich geringer ist als bei der Vergleichsbasiskomponente.

Beim Versuch, die auf +10° C temperierte Vergleichsbasiskomponente mit einem automatischen Mischgerät zu verarbeiten, kam es bei Verarbeitung der derart temperierten. Komponente zum Ausfall des Gerätes. Dagegen waren die auf +10° C temperierten Basiskomponenten B1 bis B6 auch bei +10° C verarbeitbar.

Die Katalysatorkomponenten K1 bis K4 zeigten eine vernachlässigbar geringe Temperatursensibilität der Viskosität.

### Tabelle 4

Werte des Gegendrucks, 15 Sekunden nach Versuchsbeginn, bestimmt mit einem Vorschub von 15 mm/min in Abhängigkeit von der Temperatur der Basiskomponente:

| **Basis** | **Gegendruck [N]; bei einer Basiskomponenten-Temperatur von** | | | | |
|---|---|---|---|---|---|
| | **+23° C** | **+10° C** | **0°C** | **-10° C** | **-20° C** |
| **B 1** | 2 | 4 | 6 | 18 | 21 |
| **B 2** | 2 | 5 | 10 | 15 | 19 |
| **B 3** | 2 | 4 | 12 | 18 | 30 |
| **B 4** | 2 | 6 | 8 | 10 | 18 |
| **B 5** | 3 | 5 | 11 | 14 | 22 |
| **B 6** | 3 | 6 | 8 | 20 | 28 |
| **VB1** | 8 | 50 | 450 | >500 | > 500 |

| | | | | | |
|---|---|---|---|---|---|
| Messbereichsgrenze = 500 N | | | | | |

Die Mischungen gemäß den Erfindungsbeispielen 1 bis 8 (Tabelle 2) führten zu Formkörpem, die nach einer Lagerzeit bei 23° C und 50 % relativer Luftfeuchtigkeit von 24 Stunden eine Shore A-Härte deutlich über 20 aufwiesen.

Die ermittelten Werte für die Reißdehnung lagen über 70 % und die Werte der Reißfestigkeit über 0,7 MPa und erfüllten damit die Anforderungen, die im allgemeinen an zweikomponentige Abformmassen auf der Basis von N-Alkylaziridinopolyethern gestellt werden.

## Patentansprüche

1. Verwendung von N-Alkylaziridinopolyethern, deren Polyetherteil 10 bis 250 Methylseitengruppen pro 1.000 C- und O-Atome besitzt, wobei die zahlenmittleren Molmassen der N-Alkylaziridinopolyether im Bereich von 1.000 bis 25.000 g/mol liegen, zur Verwendung der Temperatursensibilität von Dentalmassen hinsichtlich Viskosität.

2. Verwendung nach Anspruch 1, wobei die Dentalmasse eine Basiskomponente und eine Katalysatorkomponente umfasst und wobei die Basiskomponente mindestens einen N-Alkylaziridinopolyether enthält, dessen Polyetherteil 10 bis 250 Methylseitengruppen pro 1.000 C- und O-Atome besitzt, wobei die zahienmittieren Molmassen der N-Alkylazidinopolyether im Bereich von 1.000 bis 25.000. g/mol liegen.

3. Verwendung nach Anspruch 2, wobei nach der Mischung von Basiskomponente und Katalysatorkomponente die aushärtende Zubereitung jeweils bezogen auf 100 Masseteile enthält:
(A) 30 bis 97 Teile von mindestens einem N-Alkylaziridinopolyether, dessen Polyetherteil 10 bis 250 Methylseitengruppen pro 1.000 C- und O-Atome besitzt, wobei die zahlenmittleren Molmassen der N-Alkylaziridnopolyether im Bereich von 1.000 bis 25.000 g/mol Regen,
(B) 1 bis 10 Teile von Startersubstanzen, die geeignet sind, die Aushärtung der N-AleazlridinopolyL-ther zu bewirken,
(C) 1 bis 35 Teils von organischen Vordünnungsmhteln,
(D) 1 bis 50 Teile von Modifikatoren.

4. Verwendung nach einem der verstehenden Ansprüche wobei die Polyetherteile der N-Alkylaziridinopolyether Homo- und Mischpolymerisate des Propylenoxids darstellen, die mindestens eine und bis zu zehn N-Alkylaziridinogruppen im Molekül enthalten.

5. Verwendung nach einem der verstehenden Ansprüche wobei der Polyetherteil der N-5 Akylazihdinapolyeter aus Homopolymerisaten des Propylenoxids mit zahlenmittleren Molmassen im Bereich von 1.000 bis 20.0000 g/mol besteht.

6. Verwendung nach einem der verstehenden Ansprüche 1 bis 4, wobei die N-Alkylaziridinopolyether hergestellt werden aus Mischpolyether-Polyolen, gebildet aus den Monomeren Propylenoxid und Ethylenoxid und/oder Tetrahydrofuran.

## Claims

1. Use of N-alkylaziridinopolyethers, the polyether moiety of which has from 10 to 250 methyl side groups per 1000 C and O atoms, the number average molar masses of the N-alkylaziridinopolyethers being in the range from 1000 to 25 000 g/mol, for reducing the temperature sensitivity of dental compounds with respect to viscosity.

2. Use according to Claim 1, the dental compound comprising a base component and a catalyst component, the base component containing at least one N-alkylaziridinopolyether, the polyether moiety of which has from 10 to 250 methyl side groups per 1000 C and O atoms, the number average molar masses of the N-alkylaziridinopolyether being in the range from 1000 to 25 000 g/mol.

3. Use according to Claim 2, after the mixing of the base component and catalyst component, the curing formulation containing, based in each case on 100 parts by mass:
(A) from 30 to 97 parts of at least one N-alkylaziridinopolyether, the polyether moiety of which has from 10 to 250 methyl side groups per 1000 C and O atoms, the number average molar masses of the N-alkylaziridinopolyether being in the range from 1000 to 25 000 g/mol,
(B) from 1 to 10 parts of initiator substances which are suitable for bringing about the curing of the N-alkylaziridinopolyethers,
(C) from 1 to 35 parts of organic diluents,
(D) from 1 to 50 parts of modifiers.

4. Use according to any of the above claims, the polyether moieties of the N-alkylaziridinopolyethers being homopolymers and copolymers of propylene oxide which contain at least one and up to ten N-alkylaziridino groups in the molecule.

5. Use according to any of the above claims, the polyether moiety of the N-alkylaziridinopolyethers consisting of homopolymers of propylene oxide having number average molar masses in the range from 1000 to 20 000 g/mol.

6. Use according to any of the above Claims 1 to 4, the N-alkylaziridinopolyethers being prepared from copolyether-polyols, formed from the monomers propylene oxide and ethylene oxide and/or tetrahydrofuran.

## Revendications

1. Utilisation de N-alkylaziridinopolyéthers dont le fragment polyéther comporte de 10 à 250 groupes latéraux méthyle pour 1 000 atomes de carbone et d'oxygène, les masses moléculaires moyennes en nombre des N-alkylaziridinopolyéthers se situant dans la plage allant de 1 000 à 25 000 g/mole, pour diminuer la sensibilité à la température de matières dentaires, eu égard à la viscosité.

2. Utilisation selon la revendication 1, dans laquelle la matière dentaire comprend un composant de base et un composant catalyseur, et dans laquelle le composant de base contient au moins un N-alkylaziridinopolyéther dont le fragment polyéther comporte de 10 à 250 groupes latéraux méthyle pour 1 000 atomes de carbone et d'oxygène, la masse moléculaire moyenne en nombre des N-alkylaziridinopolyéthers se situant dans la plage allant de 1 0000 à 25 000 g/mole.

3. Utilisation selon la revendication 2, dans laquelle, après le mélange du composant de base et du composant catalyseur, la préparation durcie contient chaque fois, par rapport à 100 parties en masse :
(A) 30 à 97 parties d'au moins un N-alkylaziridinopolyéther dont le fragment polyéther comprend de 10 à 250 groupes latéraux méthyle pour 1 000 atomes de carbone et d'oxygène, la masse moléculaire moyenne en nombre des N-alkylaziridinopolyéthers se situant dans la plage allant de 1 000 à 25 000 g/mole,
(B) 1 à 10 parties de substances d'amorçage, qui sont appropriées à provoquer le durcissement des N-alkylaziridinopolyéthers,
(C) 1 à 35 parties de diluants organiques,
(D) 1 à 50 parties de modificateurs.

4. Utilisation selon l'une quelconque des revendications précédentes, dans laquelle les fragments polyéther des N-alkylaziridinopolyéthers représentent des homo- et copolymères de l'oxyde de propylène qui contiennent dans la molécule au moins un et jusqu'à 10 groupes N-alkylaziridino.

5. Utilisation selon l'une quelconque des revendications précédentes, dans laquelle le fragment polyéther des N-alkylaziridinopolyéthers consiste en homopolymères de l'oxyde de propylène à masses moléculaires moyennes en nombre dans la plage de 1 000 à 20 000 g/mole.

6. Utilisation selon l'une quelconque des revendications 1 à 4, dans laquelle les N-alkylaziridinopolyéthers sont préparés à partir de copolyétherpolyols, formés à partir des monomères oxyde de propylène et oxyde d'éthylène et/ou tétrahydrofuranne.
